# EUROPEAN PATENT APPLICATION

(11) **EP 3 561 482 A1**
(43) Date of publication of application: **30.10.2019**
(21) Application number: 19168990.0
(22) Date of filing: 12.04.2019
(51) Int. Cl.: G01N 15/14, G01F 1/36, G01F 1/74

(54) **FLOW MEASUREMENT IN A SENSOR DEVICE THAT COMPRISES AN ENVIRONMENTAL SENSOR**

(71) Applicant: Sensirion AG, 8712 Stäfa (CH)
(72) Inventor: Krähenbühl, Thomas, 8712 Stäfa (CH); Kostner, Stefan, 8712 Stäfa (CH); Hornung, Mark, 8712 Stäfa (CH); Fuhrer, Samuel, 8712 Stäfa (CH)
(74) Representative: Detken, Andreas

(57) **Abstract**

A sensor device comprises an inlet (11), an outlet (12), and a main channel (13) extending between the inlet (11) and the outlet (12) so as to allow a fluid flow through the main channel (13) along a flow direction (F). An environmental sensor (20) is arranged to interact with a fluid in a measurement region (14) of the main channel. The main channel has a first pressure tap (31) upstream from the measurement region and a second pressure tap (32) downstream from the measurement region. A differential pressure sensor (40) determines a pressure difference between the first and second pressure taps.

## Description

### TECHNICAL FIELD

The present invention relates to a sensor device comprising a main channel extending between an inlet and an outlet of the sensor device and comprising an environmental sensor configured to determine at least one property of a fluid in a measurement region of the main channel. The environmental sensor can be, e.g., a particulate matter sensor or a gas sensor for determining the concentration of one or more gaseous analytes in air.

### PRIOR ART

In such a sensor device the output signals of the environmental sensor will often not only depend on the composition of the fluid that enters the main channel, but also on the flow rate through the main channel. It is therefore desirable to determine the flow rate through the main channel.

WO 2018/100209 A2 discloses a particulate matter sensor device comprising an enclosure that delimits a flow channel. A radiation source emits radiation into the flow channel. A radiation detector detects at least part of the radiation after its interaction with particulate matter in a fluid sample that flows through the flow channel. The device can comprise a flow meter for determining a flow rate in the flow channel. The document is silent about the type and configuration of the flow meter.

Utility model CN 206235536 U discloses a particulate matter sensor device comprising an air passage. A laser shines light into the air passage, and a photodetector detects light scattered from particles suspended in the air. A thermal flow meter is provided for measuring the air flow rate in the air passage. The flow meter is disposed on a printed circuit board. The utility model is silent about the type and configuration of the flow meter.

### SUMMARY OF THE INVENTION

It is an object of the present invention to provide a sensor device having a main channel and an environmental sensor configured to determine at least one measured variable of a fluid in a measurement region of the main channel, the sensor device being specifically configured for taking into account the effects of varying flow rates through the main channel onto the measured variable in a simple and reliable manner.

This object is achieved by an environmental sensor device according to claim 1. Further embodiments of the invention are laid down in the dependent claims.

The present invention provides a sensor device that is configured to determine at least one property a fluid. The fluid can be a gas (the term "gas" including mixtures of gases) or an aerosol, i.e., a dispersion of fine solid particles or liquid droplets in a gas. In particular, the fluid can be ambient air from the environment of the sensor device. The sensor device comprises an inlet, an outlet, and a main channel extending between the inlet and the outlet so as to allow a flow of the fluid through the main channel from the inlet to the outlet along a flow direction. The sensor device further comprises an environmental sensor that is configured to determine at least one measured variable from the fluid in a measurement region of the main channel. The main channel has a first pressure tap upstream from the measurement region and a second pressure tap downstream from the measurement region. A differential pressure sensor, preferably a flow-type differential pressure sensor, is configured to determine a pressure difference between the first and second pressure taps. A control device is configured to receive input signals and to derive output signals based on the input signals. The input signals include environmental sensor signals from the environmental sensor and differential pressure signals from the differential pressure sensor. Specifically, the output signals depend on both the environmental sensor signals and the differential pressure signals. The output signals are a measure for said at least one property of the fluid.

A flow through the main channel will cause a pressure difference between the first and second pressure taps due to the flow resistance in the main channel between the first and second pressure taps, the magnitude of the pressure difference depending on the flow rate through the main channel. By determining the pressure difference between the pressure taps, a measure for the flow rate in the main channel is obtained, and output signals that take the flow rate appropriately into account can be derived.

The first and second pressure taps are arranged upstream and downstream of the measurement region, respectively. Accordingly, the pressure difference is determined in a "bypass" configuration, the differential pressure sensor bypassing the measurement region. This configuration provides much flexibility for tailoring the design of the main channel and the differential pressure sensor to various scenarios with vastly different flow rates, without changing the principal design of the sensor device.

The differential pressure sensor can be a flow-type differential pressure sensor. A flow-type differential pressure sensor comprises first and second sensor ports and a (typically narrow) flow channel between the sensor ports. A pressure difference between the sensor ports causes a small flow through the flow channel. A microthermal sensor determines a measure for the flow rate of this small flow. Thereby in essence a flow rate through a bypass channel that bypasses the measurement region is measured. The flow rate in the bypass channel is much lower than in the main channel. A flow-type differential pressure sensor in a bypass configuration is therefore less susceptible to contamination from dust, water, snow, etc. than a thermal flow sensor that is directly present in the main channel.

The terms "upstream" and "downstream" are to be understood in respect to a flow from the inlet to the outlet. In other words, the first pressure tap, which is arranged upstream from the measurement region, is located between the inlet and the measurement region. Likewise, the second pressure tap, which is arranged downstream from the measurement region, is located between the measurement region and the outlet. Each pressure tap can be formed by a (typically small) opening in a wall that delimits the main channel.

In the present disclosure, the term "environmental sensor" is to be understood as relating to any arrangement of one or more components that are configured to determine one or more measured variables from a fluid that has entered the main channel of the sensor device from the environment. In particular, the fluid can be ambient air, and the sensor device can be designed to monitor air quality, e.g., air outdoors near a road or indoors in a closed room, air entering an HVAC system, air inside a car or entering the interior of the car, air inside a ship, air inside a plane, etc. In some embodiments, the environmental sensor can include a particulate matter sensor that is configured to determine one or more properties of particulate matter (PM) in an aerosol. In other embodiments, the environmental sensor can include a gas sensor that is configured to determine the concentration of one or more gaseous analytes in air, such as volatile organic compounds (VOCs), nitrogen oxide (NOx), carbon monoxide (CO) or carbon dioxide (CO₂). In other embodiments, the environmental sensor can include a humidity sensor for determining the humidity of air or another type of sensor for determining any other quantity that characterizes air quality.

Generally speaking, when an environmental sensors is employed, one is often interested in the concentration of some constituent of the fluid, and accordingly, the at least one property of the fluid that is determined by the sensor device can include a concentration of a constituent of the fluid per unit volume.

In particular, the environmental sensor can comprise a particulate matter sensor for determining at least one measured variable that is related to particulate matter in an aerosol. Specifically, the particulate matter sensor can comprise a radiation source configured to create radiation in the main channel and a radiation detector configured to detect radiation that is scattered from particulate matter in the fluid in the measurement region. The radiation source can be a light source for creating visible, IR or UV light. In particular, the light source can comprise a laser or LED and optionally one or more lenses for focusing the light from the laser/LED into the measurement region of the main channel. The radiation detector can be a photodetector arranged to register scattered light. Such a particulate matter sensor typically works as a particle counter, i.e., it registers individual scattering events and optionally the magnitude of each scattering event. Thereby the number of particles that are detected per unit time can be counted, i.e., the at least one measured variable includes a number of particles per unit time, and optionally their size distribution can be determined or a size cutoff can be defined. The number per unit time depends heavily on the flow rate through the main channel. Often, however, one is not primarily interested in the number per unit time, but rather in the number or mass concentration per unit volume of the fluid, i.e., the at least one property of the fluid that is to be determined by the sensor device includes a concentration of the particulate matter per unit volume. In order to be able to determine a concentration per unit volume from the number per unit time, a measure for the volumetric flow rate of the fluid is required. The present invention enables an independent determination of a measure for the volumetric flow rate by determining the pressure difference across the measurement region. In particular, the control device can be configured to derive volumetric flow rate signals based on the differential pressure signals using calibration data, and to calculate the output signals from the environmental sensor signals and the volumetric flow rate signals, e.g., by a simple division.

In other applications, the measured variable may already be a direct measure of a concentration. This is the case, e.g., with many gas sensors for detecting one or more gaseous analytes in air, or with many humidity sensors. However, even in such cases the measured variable will often to some extent not only depend on the composition of the fluid that enters the main channel, but also on the flow rate through the main channel. This may, for instance, be due to cooling or dehydrating effects of the air flow, which may influence the sensor signals. Therefore, even for sensors that directly determine a concentration measure, it is therefore desirable to have a measure for the flow rate through the main channel in order to be able to correct the sensor signals for flow effects. The present invention enables such corrections, and accordingly the at least one property of the fluid to be determined by the gas sensor can include a flow-corrected concentration value and/or flow-corrected humidity value.

In some prior-art implementations, the flow rate is estimated from some other known parameters. For instance, a forced flow through the main channel may be created by a fan, and the power that is supplied to the fan may be used as a measure for the resulting flow rate. However, the actual flow rate can be influenced by a variety of other parameters than by the power level that is supplied to the fan, like ambient pressure and temperature, and it can be influenced by effects like aging of the fan and contaminations in the flow channel. If the flow through the main channel is created by other means than by a fan, the flow rate might not even be known at all. For instance, a variable flow rate might be created by a blower in a HVAC system or by the influence of headwind in a vehicle, and no estimate of the flow rate might be available. The present invention makes an improvement over such implementations by enabling an independent determination of a measure for the flow rate through the main channel.

The present invention also enables the detection of congestions of the main channel. This can be achieved by correlating the measured pressure difference at different flow rates with other quantities that correlate with the flow rate in the main channel. For instance, if the sensor device comprises a fan, the pressure difference can be correlated with fan speed or fan power. The dependence of the pressure difference on the fan speed or fan power will change in the presence of congestions of the main channel. Such changes can readily be detected.

Generally the pressure difference between the pressure ports not only depends on the volumetric flow rate through the main channel, but also on the temperature and the absolute pressure of the fluid that enters the flow channel. This is in particular true if the fluid is a gas or aerosol, such as air, since the fluid density will then depend on the temperature and pressure through the general gas equation. In simple embodiments, for obtaining the volumetric flow rate from the pressure difference, simply an assumption of some fixed temperature and pressure can be made, e.g., using a known average operating temperature of the sensor device and using the average air pressure at sea level. In more sophisticated embodiments, the sensor device further comprises a temperature sensor, and the control device is configured to receive temperature signals from the temperature sensor. The input signals to the control device thus include the temperature signals, and the control device can be configured to take into account the temperature signals when it derives the output signals. Thereby a better estimate of the property of the fluid can be obtained than by just taking into account the differential pressure signals. In particular, the control device can be configured to determine temperature-corrected volumetric flow rate signals based on the differential pressure signals and the temperature signals, and to determine the output signals based on the measured quantity and the temperature-corrected volumetric flow rate signals. If the property to be determined by the sensor device is a concentration, temperature correction can alternatively be carried out as follows: The control device can be configured to determine uncorrected concentration signals from the environmental sensor signals and the differential pressure signals under an assumption of some fixed temperature. The control device can be configured to subsequently correct these uncorrected concentration signals using the temperature signals. The output signals can include the uncorrected and/or corrected concentration signals.

The temperature sensor can be a separate unit, which can be mounted on a common circuit board with the differential pressure sensor, it can be integrated in a common housing with the differential pressure sensor, or it can even be integrated on the same semiconductor die as the differential pressure sensor.

If the environmental sensor device comprises a radiation source, the control device can be configured to control a radiation power level at which the radiation source is operated, taking into account the input signals, in particular, the differential pressure signals and, optionally, temperature signals from a temperature sensor. In this manner the radiation power can be adapted to different flow rates through the main channel.

The sensor device can further comprise a fan. The fan can advantageously be arranged downstream from the second pressure tap. The control device can be configured to control a fan power level at which the fan is operated, taking into account the input signals, in particular, the differential pressure signals and, optionally, temperature signals from a temperature sensor. In this manner the flow rate in the main channel can be regulated.

In order to increase the pressure difference between the first and second pressure taps that is caused by a flow through the main channel, the sensor device can comprise a flow restrictor or a constriction in the main channel between the first and second pressure taps. If a flow restrictor is used, the sensor device can easily be adapted to various ranges of flow rates by choosing a corresponding flow restrictor, without having to change the overall configuration of the sensor device. If a constriction is present, the constriction can be present in the measurement region or nearby this region, in particular, closely upstream of this region. In particular, the constriction can be configured to modify (in particular, accelerate) the flow in the measurement region so as to minimize particulate matter deposition on the components of the environmental sensor arrangement, as proposed in WO 2018/100209 A2.

The flow channel can be bent between the first and second sensor taps. A bend will also cause an increase of the pressure difference between the first and second pressure taps, since a deflection of the flow will cause a corresponding backpressure. In particular, the main channel can be generally U- or L-shaped.

In the case of a generally U-shaped flow channel, the flow channel can have an inlet section along the first leg of the U, an intermediate section that connects the two legs of the U, and an outlet section along the second leg of the U. In consequence, the flow direction in the outlet section will be essentially antiparallel to the flow direction in the inlet section, and the intermediate section connects a downstream end of the inlet section with an upstream end of the outlet section. The measurement region is then advantageously located in the intermediate section (i.e., in the section that connects the two legs of the U), the first pressure tap is located in the inlet section (i.e., in the first leg of the U), and the second pressure tap is located in the outlet section (i.e., in the second leg of the U). The differential pressure sensor is preferably arranged between the inlet and outlet sections (i.e., inside the U between its two legs). If the environmental sensor implements a particulate matter sensor, it may comprise a radiation source, as already discussed above. Some radiation sources require a considerable amount of space. In order to easily accommodate both the radiation source and the differential pressure sensor in the sensor device, the radiation source is preferably arranged opposite the differential pressure sensor with respect to the measurement region. In other words, the radiation source is preferably arranged outside the U adjacent to the intermediate section.

As already discussed, the differential pressure sensor can itself have first and second sensor ports, the differential pressure sensor being configured to determine the pressure difference between the first and second sensor ports. The first sensor port is then in fluid communication with the first pressure tap, and the second sensor port is in fluid communication with the second pressure tap. To this end, the sensor device can comprise a sensor inlet channel that branches off from the main channel at the first pressure tap, the sensor inlet channel extending from the first pressure tap to the first sensor port of the differential pressure sensor. Likewise, the sensor device can comprise a sensor outlet channel that opens out into the main channel at the second pressure tap, the sensor outlet channel extending from the second sensor port of the differential pressure sensor to the second pressure tap. The sensor inlet and outlet channels can have the same or different lengths. For instance, the sensor inlet channel can be longer than the sensor outlet channel or vice versa.

In order to minimize contamination of the differential pressure sensor with particulate matter that may be carried by the fluid flow through the main channel, it is advantageous to configure the sensor inlet channel in such a manner that particulate matter would have to change its main direction of movement (i.e., the direction of its momentum) if it were to enter the sensor inlet channel. To this end, the sensor inlet channel, in a region adjacent to the first pressure tap, advantageously extends at an angle of at least 120°, preferably at least 135°, relative to the flow direction in the main channel at the first pressure tap.

In advantageous embodiments that enable simple assembly and disassembly of the sensor device, the differential pressure sensor and at least one component of the environmental sensor (in particular, in the case of a particulate matter sensor, the radiation detector and/or radiation source) are mounted on a common circuit board. The control device can be implemented as a microcontroller. Optionally also the control device is mounted on the common circuit board.

In order to enable rapid assembly and easy servicing of a sensor device that comprises a particulate matter sensor, the sensor device can be constructed as follows: The sensor device comprises an upper enclosure element and a lower enclosure element arranged below the upper enclosure element and connected to the upper enclosure element in a fluid-tight manner. The upper and lower enclosure elements are configured so as to together delimit ("enclose") the main channel. A circuit board is arranged at the bottom side of the lower enclosure element, which bottom side faces away from the upper enclosure element. The differential pressure sensor and the radiation detector are mounted on the circuit board. The lower enclosure element has first and second pressure sensor openings into which the first and second sensor ports of the differential pressure sensor are inserted so as to be in fluid communication with the first and second pressure taps, respectively. To this end, the first and second sensor ports are preferably nipple-shaped and point upwards, i.e., towards the lower enclosure element. The lower enclosure element further has a radiation detector opening, the radiation detector being received in or below the radiation detector opening. The radiation source is preferably held between the upper and lower enclosure elements, the lower enclosure element preferably having a radiation source opening for electrically connecting the radiation source to the circuit board, preferably via a plug-in connection. In this manner, the sensor device can be very easily assembled and disassembled.

The present invention also provides a method of operating the sensor device of the present disclosure. The method comprises:
receiving environmental sensor signals from the environmental sensor arrangement;
receiving differential pressure signals from the differential pressure sensor;
optionally receiving temperature signals from a temperature sensor of the sensor device; and
deriving output signals that are a measure for the at least one property of the fluid, based on the environmental sensor signals, the differential pressure signals, and optionally the temperature signals.

The output signals can include at least one of the following:
output signals that are a measure for a concentration of particulate matter in the fluid;
output signals that are a flow-corrected measure for a size distribution of particulate matter in the fluid;
output signals that are a flow-corrected measure for a concentration of one or more gaseous analytes in air, in particular, of volatile organic compounds (VOCs), nitrogen oxides (NOx), carbon monoxide (CO) and/or carbon dioxide (CO₂); and
output signals that are a flow-corrected measure for a humidity of the fluid.

The output signals can be derived using calibration data, which can be stored in a memory of the control device in the form of one or more lookup tables.

The method can further comprise determining control signals, using the differential pressure signals and optionally the temperature signals, the control signals comprising:
radiation power control signals for controlling a radiation power level at which a radiation source in the sensor device is operated; and/or
fan power control signals for controlling a fan power level at which a fan is operated.

The method can further comprise deriving and outputting volumetric flow rate signals that are indicative of a volumetric flow rate in the main channel.

The present invention further provides a computer program product comprising computer program instructions that, when executed in a processor of the control device, cause the control device to carry out the above method. The computer program may be stored in a storage medium, in particular, on a non-volatile data carrier, or it may be provided for download via a network.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a highly schematic view of a sensor device according to a first embodiment;
- Fig. 2: shows an enlarged detail view of a portion of the sensor device in Fig. 1, illustrating the configuration at the first pressure tap;
- Fig. 3: shows a functional diagram illustrating the arrangement and interaction of the various components of the sensor device in Fig. 1;
- Fig. 4: shows a highly schematic view of a sensor device according to a second embodiment;
- Fig. 5: shows a highly schematic view of a sensor device according to a third embodiment;
- Fig. 6: shows a functional diagram illustrating the arrangement and interaction of the various components of the sensor device in Fig. 5;
- Fig. 7: shows an exploded view of a sensor device according to a fourth embodiment;
- Fig. 8: shows a top plan view of the lower enclosure element of the sensor device in Fig. 7;
- Fig. 9: shows a highly schematic block diagram of an exemplary control device; and
- Fig. 10: shows a flow diagram illustrating a possible method of operating a sensor device.

### DESCRIPTION OF PREFERRED EMBODIMENTS

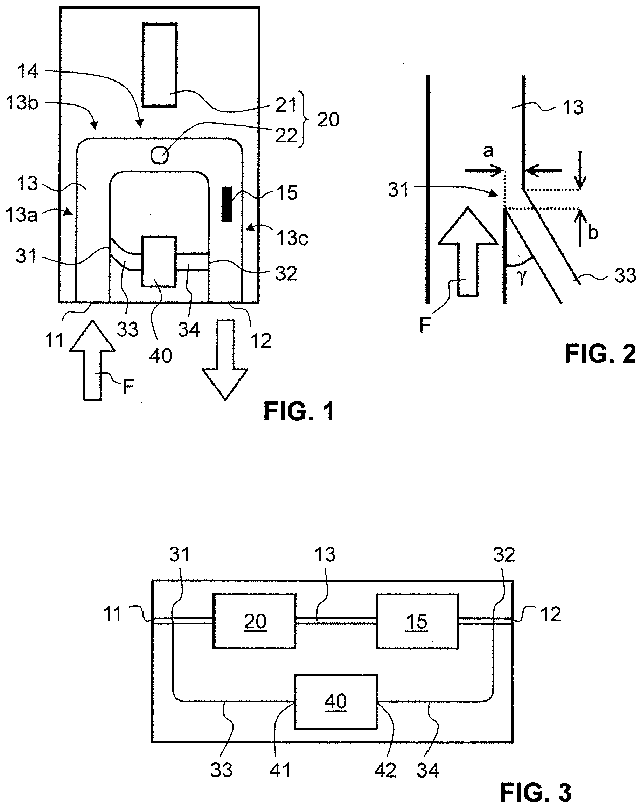

Fig. 1 shows, in a highly schematic manner, a sensor device according to an embodiment of the present invention. The sensor device defines an inlet 11 and an outlet 12 for a fluid flow. A generally U-shaped main channel 13 extends from the inlet 11 to the outlet 12. An aerosol sample is passed through the flow channel along a flow direction F.

The generally U-shaped main channel 13 has three sections: an inlet section 13a, an intermediate section 13b, and an outlet section 13c. The inlet section 13a extends along the first leg of the U, leading from the inlet 11 to the intermediate section 13b. The outlet section 13c extends along the second leg of the U, leading from the intermediate section 13b to the outlet 12. The flow direction in the outlet section 13c is opposite to the flow direction in the inlet section 13a. The intermediate section 13b connects the inlet section 13a and the outlet section 13b at their respective ends. The flow direction in the intermediate section 13b is generally transverse to the flow direction in the inlet and outlet sections 13a, 13c, respectively.

An environmental sensor arrangement 20 for determining a particulate matter (PM) concentration of the aerosol sample is arranged adjacent to the intermediate section 13b of the main channel 13. The environmental sensor arrangement 20 comprises a radiation source in the form of a laser 21 and a radiation detector in the form of a photodetector 22. The laser 21 creates a laser beam, which enters a measurement region 14 in the intermediate section 13b of the main channel 13. The laser 21 is arranged adjacent to the intermediate section 13b away from the inlet and outlet sections 13a, 13c, i.e., outside of the U. The photodetector 22 is arranged adjacent to the measurement region 14, its sensitive area having a surface normal that extends at an angle of 90° relative to the laser beam such that the laser beam does not directly impinge onto the photodetector 22. The photodetector 22 registers light that is scattered from particulate matter present in the aerosol sample in the measurement region. The intensity of the light that is scattered from each particle correlates with the size of the particle. By counting scattering events from single particles, the number of the particles in the aerosol that are registered by the photodetector per unit time can be determined. By analyzing the intensity distribution of the signals from the photodetector, the size distribution of the particles can be estimated.

The sensor device in Fig. 1 further comprises a differential pressure sensor 40, which measures a pressure difference between a first pressure tap 31 upstream from the measurement region 14 and a second pressure tap 32 downstream from the measurement region 14. Each pressure tap (pressure port) is formed by a small opening in the wall that delimits the main channel 13. The first pressure tap 31 is located in the inlet section 13a of the main channel, while the second pressure tap 32 is located in the outlet section 13c. The differential pressure sensor 40 is disposed between the inlet and outlet sections 13a, 13c, i.e., between the two legs of the U, opposite the laser 21 with respect to the intermediate section 13b. The differential pressure sensor 40 has two sensor ports, which are connected to the pressure taps via a sensor inlet channel 33 and a sensor outlet channel 34, respectively.

A flow through the main channel 13 causes a pressure difference between the first and second pressure taps 31, 32 due to the flow resistance in the main channel 13. In order to increase the pressure difference for a given flow rate, a flow restrictor 15 is placed in the main channel 13 downstream from the measurement region 14. By measuring the pressure difference using the differential pressure sensor 40, the flow rate through the main channel 13 can be determined. The flow resistance of the flow restrictor 15 can be chosen in such a manner that the expected dynamic range of the flow rates through the main channel matches the dynamic range of the differential pressure sensor.

As illustrated in Fig. 2, the sensor inlet channel 33 is configured in a specific manner so as to minimize the amount of particulate matter that reaches the differential pressure sensor 40. In particular, the sensor inlet channel 33 is configured in such a manner that particles in the fluid flow would have to change their direction of movement (or, equivalently, the direction of their momentum) if they were to enter the sensor inlet channel 33. To this end, the sensor inlet channel 33 branches off from the main channel 13 at an angle of more than 90° relative to the flow direction F at the first pressure tap 31. In Fig. 2, the angle at which the sensor inlet channel 33 branches off is 180°-γ, wherein the tap angle γ is less than 90°, preferably not more than 60° and in particular not more than 45°. The pressure tap 31 is relatively small compared to the cross section of the main channel 31: The cross-sectional area of the sensor inlet channel 33 is preferably smaller than the cross-sectional area of the main channel at least by a factor of five. Thereby the entry of excessive amounts of fluid into the sensor inlet channel is avoided. The inner channel wall surface immediately downstream from the pressure tap 31 may be laterally slightly offset outwardly from the wall surface immediately upstream from the pressure tap 31 in order to reduce particle contamination around and inside the first pressure tap 31. The lateral offset "a" should however not be too big, being preferably smaller than the size "b" of the pressure tap 31 so as to limit the influence of the Bernoulli effect on the pressure at the first pressure tap 31.

Fig. 3 shows a highly schematic functional diagram illustrating the arrangement and interaction of the various components of the sensor device in Fig. 1. The main flow channel 13 extends from the inlet 11 to the outlet 12. The environmental sensor arrangement 20 is arranged adjacent to the flow channel 13 upstream from the flow restrictor 15. The first pressure tap 31 is arranged upstream from the environmental sensor arrangement 20, while the second pressure tap 32 is arranged downstream from the flow restrictor 15. The sensor inlet channel 33 extends from the first pressure tap 31 to the first sensor port 41 of the differential pressure sensor 40, while the sensor outlet channel 34 extends from the second sensor port 42 of the differential pressure sensor 40 to the second pressure tap 32.

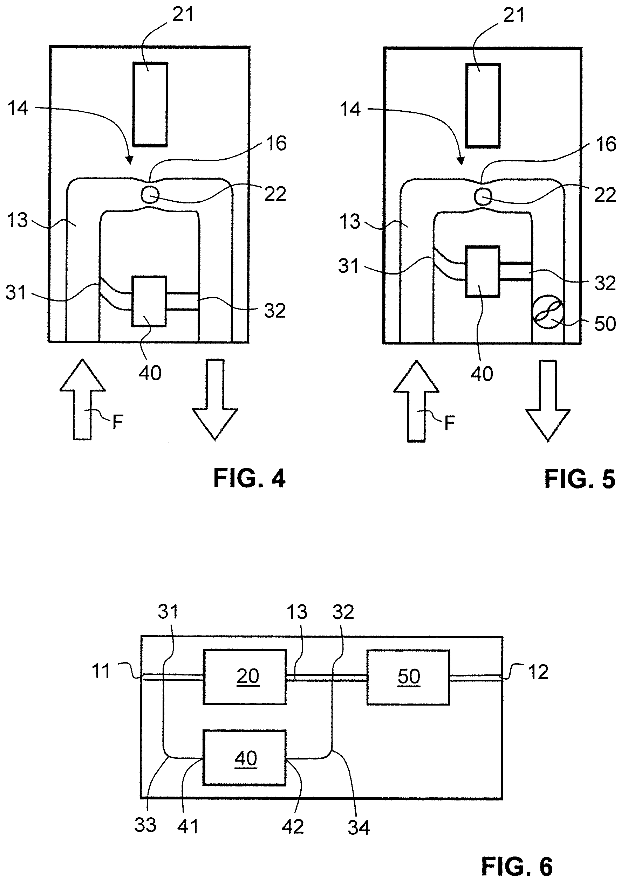

As illustrated in Fig. 4, instead of providing a flow restrictor 15, the main channel 13 can be provided with a constriction 16. The constriction 16 can be arranged in the measurement region 14 of the main channel 13, accelerating the fluid flow in the measurement region 14 so as to minimize contamination of the laser 21 and the photodetector 22 by the particulate matter in the aerosol sample.

As illustrated in Fig. 5, the sensor device can further comprise a fan 50. The fan 50 is advantageously arranged downstream from the second pressure tap 32 (or upstream from the first pressure tap 31). In this manner the pressure difference across the fan, which generally depends not only on the flow rate, but also on other parameters like fan speed, is not reflected in the reading of the differential pressure sensor 40.

Fig. 6 shows a corresponding functional diagram of the arrangement and interaction of the various components of the sensor device in Fig. 5. As apparent from Fig. 6, the fan is arranged between the second pressure tap 32 and the outlet 12.

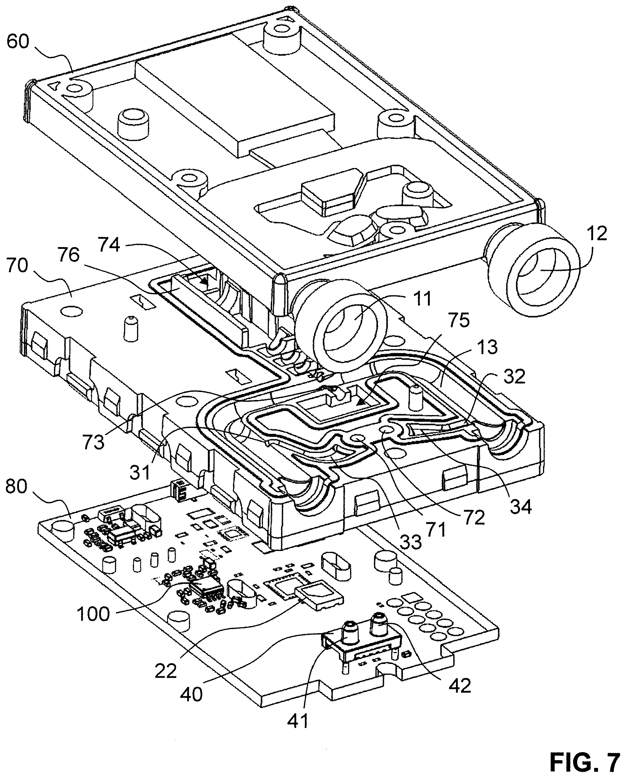

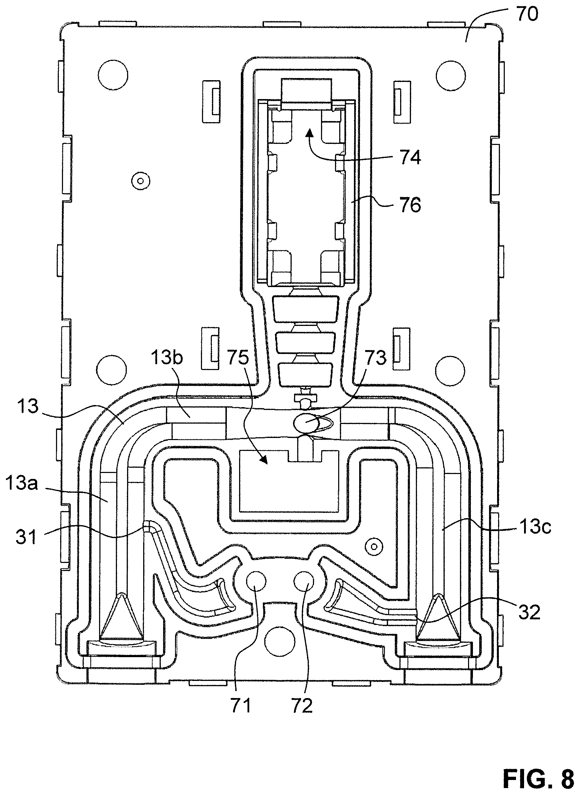

Figures 7 and 8 illustrate a possible concrete design of a sensor device according to the present invention. A U-shaped main channel 13 is formed between an upper enclosure element 60 and a lower enclosure element 70, which together enclose the main channel 13. Each of the enclosure elements is made of molded plastic. An inlet 11 and an outlet 12 are formed by two short tubular structures held between the enclosure elements 60, 70. A first pressure tap 31 is formed in an inlet section 13a (see Fig. 8) of the main channel 13, approximately halfway along the length of the first leg of the U. A second pressure tap 32 is formed in an outlet section 13c of the main channel 13, closely upstream from the outlet 12 in the second leg of the U. Each pressure tap takes the form of a small opening in the circumferential wall that defines the main channel 13. Each pressure tap is delimited partly by the upper enclosure element 60 and partly by the lower enclosure element 70.

A sensor inlet channel 33 extends from the first pressure tap 31 to a first pressure sensor opening 71 in the lower enclosure element 70. A sensor outlet channel 34 extends from a second pressure sensor opening 72 in the lower enclosure element 70 to the second pressure tap 32. The pressure sensor openings 71, 72 are arranged in the center of the U, between its two legs. They are sized to receive first and second sensor ports 41, 42 of a differential pressure sensor 40. Each sensor port 41, 42 has the shape of a nipple that extends upwards from the main body of the differential pressure sensor 40.

The differential pressure sensor 40 is preferably a flow-based differential pressure sensor. This type of differential pressure sensor defines a narrow flow channel between the two sensor ports 41, 42. A thermal flow sensor is arranged adjacent the flow channel. The thermal flow sensor comprises a heater and two temperature sensors on both sides of the heater along the flow channel. A pressure difference between the sensor ports 41, 42 causes a small flow through the flow channel, the flow velocity depending on the pressure difference. When the heater is operated, the flow will cause the temperatures measured by the temperature sensors to be different. From the temperature difference, the flow velocity and therefore the pressure difference can be determined. In particular, the differential pressure sensor 40 can be configured in the same manner as the flow sensor disclosed in US 10,151,612. The sensor inlet and outlet channels 33, 34 are sized so as to present a negligible flow resistance to the flow through the differential pressure sensor 40, i.e., their cross sectional area is significantly larger than the cross sectional area of the flow channel inside the differential pressure sensor 40.

A printed circuit board (PCB) 80 is arranged at the bottom of the lower enclosure element 70. The PCB is sized to be received in a downwardly open cavity in the bottom of the lower enclosure element 70, the cavity being delimited by downwardly extending side walls of the lower enclosure element 70. The differential pressure sensor 80 is mounted on the PCB 80. Also mounted on the PCB 80 are a photodetector 22, a control device in the form of a microcontroller 100, and various other electronic components, e.g., for voltage stabilization and for communication with the outside world. The photodetector 22 has an upwards-pointing sensitive surface. The sensitive surface is arranged below a radiation detector opening 73 in the lower enclosure element 70.

The lower enclosure element 70 has a holding structure 76 for holding the laser and one or more lenses between the upper and lower enclosure elements. A radiation source opening 74 provides access to the laser from below, enabling a plug-in electrical connection between the laser and the PCB 80 to be made. The lower enclosure element 70 further has a beam stopper opening 75 for receiving a beam stopper structure for absorbing the laser beam to avoid stray light.

The sensor device can be assembled easily by positioning the laser in the holding structure 76 of the lower enclosure element 70. The enclosure is then closed by mounting the upper enclosure element 60 on the lower enclosure element 70. Subsequently PCB 80 is pushed into the cavity in the bottom of the lower enclosure element 70. Thereby, the sensor ports 41, 42 of the differential pressure sensor 40 are pressed into the pressure sensor openings 71, 72, the photodetector is placed below the photodetector opening 73 of the lower enclosure element 70, and the laser is electrically connected to the PCB by a plug-in connection. The assembly is now ready to be placed in a protective housing.

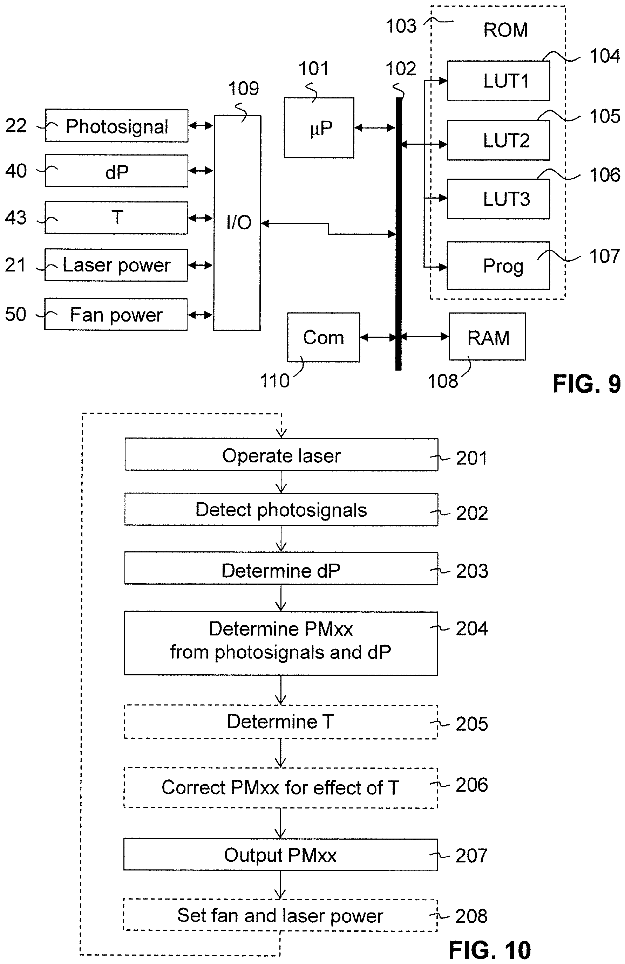

Fig. 9 illustrates, in a highly schematic manner, an exemplary functional diagram of the microcontroller 100. The microcontroller 100 comprises a microprocessor (µP) 101, which communicates with various other components via a bus 102. Also connected to the bus 102 are a read-only memory (ROM) device 103 and a random-access memory (RAM) device 108. An input/output (I/O) interface 109 connects the microcontroller 100 to various input and output devices, in particular, to the photodetector 22, to the differential pressure sensor 40, to an optional temperature sensor 43 that is integrated into the differential pressure sensor 40, to the laser 21, and to a fan 50. A communication interface 110 provides wired or wireless communication capabilities to the control device for communication with other devices, e.g., via an I²C or UART interface.

Via the I/O interface 109, the microcontroller 100 obtains photosignals from photodetector 22, differential pressure signals dP from differential pressure sensor 40, and optionally temperature signals T from temperature sensor 43. It derives output signals based on these signals. The output signals include signals that are a measure of the number concentration of particulate matter in the flow. To this end, the microcontroller 100 counts the photosignals per unit of time. The ROM device 103 stores, inter alia, a first look-up table (LUT1) 104, which contains calibration data that relate volumetric flow rate values of the fluid that flows through the main channel to the corresponding differential pressure values dP, assuming a fixed fluid density (i.e., fixed fluid pressure and temperature). By dividing the photosignal count per unit of time (i.e., the particle number per unit of time) by the volumetric flow rate, the particle number concentration is obtained up to a constant calibration factor (which may be already included in the calibration data in LUT1). An optional second lookup table (LUT2) 105 contains calibration data that relate correction factors for the volumetric flow rate (or, equivalently, for the number concentration) as determined with the aid of LUT1 to the temperature signals T, so as to correct the volumetric flow rate and/or number concentration for deviations of the actual temperature from the fixed temperature that was used for LUT1. The corrected number concentration and, optionally, volumetric flow rate are then outputted. Optional further lookup tables (LUT3) 106 may contain calibration data for other purposes, e.g., e.g. calibration data that relate particle mass or size to signal magnitude of the photosignals for converting the number concentration into a mass concentration or for determining a size distribution from the distribution of the magnitudes of the photosignals, etc. The ROM device 103 further stores program data (Prog) 107 with instructions for the microprocessor 101.

The microcontroller 100 further determines a laser power level for laser 21 and a fan power level for fan 50 based on the photosignals, differential pressure signals dP and/or temperature signals T. For instance, if the photosignals indicate an excessive number of scattering events per unit time, or if the flow rate signals indicate excessive flow, the microcontroller may cause the fan to operate at reduced power. As another example, if the photosignals indicate scattering events with excessive intensity, the microcontroller may cause the laser to operate at reduced power. As yet another example, if the temperature signals indicate excessive temperature inside the sensor device, the microcontroller may cause the fan to operate at higher power to provide additional cooling, and it may cause the laser to operate at reduced power. Many variations of the control scheme are conceivable.

An exemplary method of operation is illustrated in Fig. 10. In step 201, the microcontroller 201 operates the laser 21. In step 202, it detects and counts the resulting photosignals from photodetector 22. In step 203, it determines differential pressure signals dP. In step 204, the microcontroller determines a volumetric flow rate and a particulate matter concentration, e.g., a PM10, PM2.5 and/or PM1 value, based on the photosignals from radiation detector 22 and the differential pressure signals dP, using lookup table LUT1, as described above, assuming a fixed temperature. Here, the expression "PMxx" indicates the concentration of particles below a certain size of xx micrometers, e.g., below 10, 2.5 or 1.0 µm. The size cutoff can be made by only taking into account photosignals having a magnitude below a certain threshold. In step 205, the microcontroller optionally determines actual temperature signals T. In step 206, the microcontroller optionally corrects the PMxx values for the actual temperature signals T, using lookup table LUT2, as described above. In step 207, the microcontroller outputs the corrected PMxx values via the communication interface 110, optionally along with the (possibly temperature-corrected) volumetric flow rate. Optionally, in step 208 the microcontroller determines an appropriate laser power level and uses this power level for the next operation of the laser 21. Optionally, the microcontroller also determines an appropriate fan power level and drives the fan 50 at this power level for the next measurement.

Various modifications are possible without leaving the scope of the present invention. For instance, the radiation source can be an LED instead of a laser. Instead of or in addition to a particulate matter sensor, a different type of environmental sensor arrangement can be present, for instance a gas sensor for determining a concentration of a gaseous analyte in air, in particular, VOCs, NOx, CO or CO₂. While a flow-based differential pressure sensor is preferred due to its high precision and large dynamic range, other type of differential pressure sensors may be used as well. Instead of or in addition to integrating a temperature sensor with the differential pressure sensor, a separate temperature sensor may be used, and the temperature sensor can be disposed in a different region of the sensor device, in particular, adjacent to the main channel. The main channel can have a different configuration than the U configuration shown. Many other modifications are possible.

**LIST OF REFERENCE SIGNS**

| | | | |
|---|---|---|---|
| 11 | inlet | 76 | holding structure |
| 12 | outlet | 80 | circuit board |
| 13 | main channel | 100 | microcontroller |
| 13a | inlet section | 101 | microprocessor (µP) |
| 13b | intermediate section | 102 | bus |
| 13c | outlet section | 103 | ROM |
| 14 | measurement region | 104 | look-up table (LUT1) |
| 15 | flow restrictor | 105 | look-up table (LUT2) |
| 16 | constriction | 106 | look-up table (LUT3) |
| 20 | sensor arrangement | 107 | program memory (Prog) |
| 21 | radiation source | 108 | RAM |
| 22 | radiation detector | 109 | I/O interface |
| 31 | pressure tap | 110 | communication interface |
| 32 | pressure tap | 201 | measurement of dP |
| 33 | sensor inlet channel | 202 | operation of laser |
| 34 | sensor outlet channel | 203 | detection of photosignal |
| 40 | differential pressure sensor | 204 | measurement of T |
| 41 | first sensor port | 205 | compensation of T |
| 42 | second sensor port | 206 | determination of flow rate |
| 43 | temperature sensor | 207 | determination of PMxx |
| 50 | fan | 208 | output of PMxx |
| 60 | upper enclosure element | dP | differential pressure |
| 70 | lower enclosure element | T | temperature |
| 71 | pressure sensor opening | a | lateral offset |
| 72 | pressure sensor opening | b | tap size |
| 73 | radiation detector opening | γ | tap angle |
| 74 | radiation source opening | F | flow direction |
| 75 | beam stopper opening | | |

## Claims

1. A sensor device for determining at least one property of a fluid, preferably a gas or an aerosol, in particular, ambient air, the sensor device comprising:
an inlet (11), an outlet (12), and a main channel (13) extending between the inlet (11) and the outlet (12) so as to allow a flow of the fluid through the main channel (13) from the inlet (11) to the outlet (12) along a flow direction (F); and
an environmental sensor (20) configured to determine at least one measured variable from the fluid in a measurement region (14) of the main channel (13),
**characterised in that**
the main channel (13) has a first pressure tap (31) upstream from the measurement region (14) and a second pressure tap (32) downstream from the measurement region (14),
the sensor device comprises a differential pressure sensor (40), preferably a flow-type differential pressure sensor, the differential pressure sensor (40) being configured to determine a pressure difference between the first and second pressure taps (31, 32), and
the sensor device comprises a control device (100) configured to receive input signals and to derive output signals based on the input signals, the input signals including environmental sensor signals from the environmental sensor (20) and differential pressure signals (dP) from the differential pressure sensor (40), and the output signals being a measure for said at least one property of the fluid.

2. The sensor device of claim 1, wherein the at least one property of the fluid includes a concentration of a constituent of the fluid per unit volume.

3. The sensor device of claim 2,
wherein the environmental sensor comprises a particulate matter sensor, in particular, a particulate matter sensor comprising a radiation source (21) configured to create radiation in the main channel and a radiation detector (22) configured to detect radiation that is scattered from particulate matter in the fluid, and
wherein the at least one property of the fluid includes a concentration of the particulate matter per unit volume.

4. The sensor device of claim 2 or 3, wherein the environmental sensor (20) comprises:
a gas sensor for determining a concentration of one or more gaseous analytes in air, in particular, of volatile organic compounds (VOCs), nitrogen oxides (NOx), carbon monoxide (CO) and/or carbon dioxide (CO₂); and/or
a humidity sensor for determining a humidity of the fluid.

5. The sensor device of any one of the preceding claims, wherein the sensor device further comprises a temperature sensor (43), wherein the control device (100) is configured to receive temperature signals (T) from the temperature sensor (43), and wherein the input signals include the temperature signals (T).

6. The sensor device of any one of the preceding claims, comprising a radiation source (21) configured to create radiation in the main channel, wherein the control device (100) is configured to control a radiation power level at which the radiation source (21) is operated, based on the input signals.

7. The sensor device of any one of the preceding claims, comprising a fan (50),
wherein the fan is arranged downstream from the second pressure tap (32), and/or
wherein the control device (100) is configured to control a fan power level at which the fan (50) is operated, based on the input signals.

8. The sensor device of any one of the preceding claims, comprising a flow restrictor (15) and/or a constriction (16) in the main channel (13) between the first and second pressure taps (31, 32), the constriction (16) optionally being formed in the measurement region (14) or closely upstream thereof.

9. The sensor device of any one of the preceding claims, wherein the main channel (13) is bent between the first and second sensor taps, in particular, wherein the main channel (13) is generally U- or L-shaped.

10. The sensor device of any one of the preceding claims, comprising a sensor inlet channel (33) that branches off from the main channel (13) at the first pressure tap (31), the sensor inlet channel (33) extending from the first pressure tap (31) to a first sensor port (41) of the differential pressure sensor (40), wherein the sensor inlet channel (33), in a region adjacent to the first pressure tap (31), extends at an angle of at least 120°, preferably at least 135° relative to the flow direction (F) in the main channel (13) at the first pressure tap (31).

11. The sensor device of any one of the preceding claims, wherein the differential pressure sensor (40), at least one component of the environmental sensor (20) and optionally the control device (100) are arranged on a common circuit board (80).

12. The sensor device of any one of the preceding claims, wherein the environmental sensor (20) comprises a radiation source (21) configured to create radiation in the main channel and a radiation detector (22) configured to detect radiation that is scattered from particulate matter in the fluid, and wherein the sensor device comprises:
an upper enclosure element (60);
a lower enclosure element (70) connected to the upper enclosure element (60) in a fluid-tight manner; and
a circuit board (80) connected to a side of the lower enclosure element (70) that faces away from the upper enclosure element,
wherein the upper and lower enclosure elements (60, 70) are shaped so as to together delimit the main channel (13),
wherein the differential pressure sensor (40) and the radiation detector (22) are mounted on the circuit board (80),
wherein the lower enclosure element (70) has first and second pressure sensor openings (71, 72) into which first and second sensor ports (41, 42) of the differential pressure sensor (40) are inserted so as to be in fluid communication with the first and second pressure taps (31, 32), respectively, the first and second sensor ports (41, 42) pointing towards the lower enclosure element (70),
wherein the lower enclosure element (70) has a radiation detector opening (73), the radiation detector (22) being arranged in or below the radiation detector opening (73), and
wherein preferably the radiation source (21) is held between the upper and lower enclosure elements (60, 70), the lower enclosure element (70) preferably having a radiation source opening (74) for electrically connecting the radiation source (21) to the circuit board (80).

13. A method of operating a sensor device according to any one of the preceding claims, the method comprising:
receiving environmental sensor signals from the environmental sensor (20);
receiving differential pressure signals (dP) from the differential pressure sensor (40);
optionally receiving temperature signals (T) from a temperature sensor (43) of the sensor device; and
deriving output signals that are a measure for the at least one property of the fluid, based on the environmental sensor signals, the differential pressure signals (dP), and optionally the temperature signals (T),
wherein preferably the output signals include at least one of the following:
output signals that are a measure for a concentration of particulate matter in the fluid;
output signals that are a flow-corrected measure for a size distribution of particulate matter in the fluid;
output signals that are a flow-corrected measure for a concentration of one or more gaseous analytes in air, in particular, of volatile organic compounds (VOCs), nitrogen oxides (NOx), carbon monoxide (CO) and/or carbon dioxide (CO₂); and
output signals that are a flow-corrected measure for a humidity of the fluid.

14. The method of claim 13, further comprising determining control signals, using the differential pressure signals (dP) and optionally the temperature signals (T), the control signals comprising:
radiation power control signals for controlling a radiation power level at which a radiation source (21) in the sensor device is operated; and/or
fan power control signals for controlling a fan power level at which a fan (50) is operated.

15. A computer program product comprising computer program instructions that, when executed in a processor of the control device (100) of the sensor device according to any one of claims 1 to 12, cause the control device (100) to carry out the method of claim 13 or 14.
